# EUROPÄISCHE PATENTSCHRIFT

(11) **EP 1 254 633 B1**
(45) Veröffentlichungstag und Bekanntmachung des Hinweises auf die Patenterteilung: **28.12.2005**
(21) Anmeldenummer: 02100427.0
(22) Anmeldetag: 30.04.2002
(51) Int. Cl.: A61B 6/03, G06T 11/00

(54) **Computertomograph**
Computer tomograph
Tomographe assisté par ordinateur

(30) Priorität: 02.05.2001 DE 10121441
(43) Veröffentlichungstag der Anmeldung: 06.11.2002
(73) Patentinhaber: Philips Intellectual Property & Standards GmbH, 20099 Hamburg (DE); Koninklijke Philips Electronics N.V., 5621 BA Eindhoven (NL)
(72) Erfinder: Grass, Michael, Dr., 52066, Aachen (DE); Köhler, Thomas, Dr., 52066, Aachen (DE); Proksa, Roland, 52066, Aachen (DE)
(74) Vertreter: Volmer, Georg

(56) Entgegenhaltungen:
- EP-A- 0 969 414
- EP-A- 0 981 995
- US-A- 5 974 110
- BESSON G: "NEW CLASSES OF HELICAL WEIGHTING ALGORITHM WITH APPLICATIONS TO FAST CT RECONSTRUCTION" MEDICAL PHYSICS, AMERICAN INSTITUTE OF PHYSICS. NEW YORK, US, Bd. 25, Nr. 8, August 1998 (1998-08), Seiten 1521-1532, XP000848574 ISSN: 0094-2405

## Beschreibung

Die Erfindung betrifft einen Computertomographen mit einer ein kegelförmiges Strahlenbündel emittierenden Strahlenquelle und einer helixförmigen Relativbewegung zwischen der Strahlenquelle und dem Untersuchungsbereich. Außerdem betrifft die Erfindung ein Computerprogramm für einen derartigen Computertomographen.

Aus der WO 9936885 (PHQ 98020) ist ein solcher Computertomograph bereits bekannt. Die Helix und eine das kegelförmige Strahlenbündel jenseits eines Untersuchungsbereichs erfassende Detektoreinheit sind dabei so bemessen, dass die Detektoreinheit sämtliche Strahlen des Strahlenbündels erfasst, die durch zwei benachbarte, der Strahlenquelle gegenüberliegende Abschnitte der Helix verlaufen oder die zwischen diesen Abschnitten verlaufen. Für die Rekonstruktion werden nur die von der Detektoreinheit gelieferten Messdaten herangezogen, die zu den Strahlen in dem so definierten Messfenster gehören.

Ein beliebiger Punkt im Untersuchungbereich wird bei seinem Eintritt in das kegelförmige Strahlenbündel aus einer Richtung bestrahlt, die der Richtung um 180° entgegengesetzt ist, aus der es beim Verlassen Strahlenbündels bestrahlt wird. Da j eder Punkt im Untersuchungbereich nur über einen Winkelbereich von exakt 180° bestrahlt wird, ist dieses Verfahren gegenüber Abtastfehlern sehr empfindlich.

Diese Empfindlichkeit ist bei einem aus der US-Appln. 09/368850 (PHD 98086) bekannten Computertomographen verringert, der sich dadurch von dem vorgenannten Computertomographen unterscheidet, dass die Messdaten, die zur Rekonstruktion herangezogen wurden, innerhalb eines Messfensters liegen, das - bezogen auf den Abstand zweier benachbarter Windungen der Helix - um den Faktor 2n + 1 größer ist, wobei n eine ganze Zahl ist, die mindestens 1 beträgt. Bei diesem Verfahren wird jeder Punkt aus dem Untersuchungsbereich aus einem Winkelbereich von (2n +1)·180° bestrahlt. Die Empfindlichkeit gegenüber Abtastfehlern ist dabei weniger ausgeprägt. Dieser Vorteil wird dadurch erkauft, dass das Untersuchungsobjekt um den Faktor 2n+1 länger bestrahlt wird (bei gleicher Umlaufgeschwindigkeit). Dies kann bei einem beweglichen Objekt zu Bewegungsunschärfen führen. Außerdem können spezifische Artefakte im rekonstruierten CT-Bild auftreten.

Aufgabe der vorliegenden Erfindung ist es, einen Computertomographen zu schaffen, bei dem die geschilderten Probleme im verringerten Ausmaß auftreten. Diese Aufgabe wird erfindungsgemäß gelöst durch einen Computertomographen mit
- einer Abtasteinheit, die eine Strahlenquelle und eine damit verbundene Detektoreinheit zur Erfassung eines von der Strahlenquelle emittierten, kegelförmigen Strahlenbündels nach dem Durchgang durch einen Untersuchungsbereich bzw. durch ein darin befindliches Objekt umfasst,
- einer Antriebsanordnung zur Erzeugung einer eine Rotation um eine Rotationsachse und einen Vorschub in Richtung parallel zur Rotationsachse umfassenden Relativbewegung in Form einer Helix zwischen der Abtasteinheit und dem Untersuchungsbereich bzw. dem Objekt,
   - wobei die Helix und die Detektoreinheit so bemessen sind, dass die Detektoreinheit gleichzeitig sämtliche Strahlen des Strahlenbündels innerhalb eines ersten Messfensters erfasst, dessen in Richtung der Rotationsachse gegeneinander versetzte Ränder durch von der Strahlenquelle ausgehende und zwei in Richtung der Rotationsachse um die Strecke (2n+1)p versetzte Abschnitte der Helix schneidende Geraden definiert sind, wobei n eine ganze Zahl ≥ 1 ist und p dem achsialen Versatz zweier benachbarter Windungen der Helix entspricht,
- und mit einer Rekonstruktionseinheit zur Rekonstruktion eines der räumlichen Verteilung der Absorption innerhalb des Untersuchungsberiches entsprechenden CT-Bildes aus den von der Detektoreinheit innerhalb des ersten Messfensters akquirierten Messdaten,
   - wobei die Messdaten, die aus Strahlen abgeleitet sind, die innerhalb eines zweiten Messfensters verlaufen, mit einem anderen Gewicht in die Rekonstruktion eingehen als die Messdaten von außerhalb des zweiten Messfensters - aber innerhalb des ersten Messfensters verlaufenden Strahlen und
      wobei in Richtung der Rotationsachse das zweite Messfenster sich in der Mitte des ersten Messfensters befindet und seine gegeneinander versetzten Ränder durch von der Strahlenquelle ausgehende und zwei in Richtung der Rotationsachse um die Strecke (2m+1)p versetzte Abschnitte der Helix schneidende Geraden definiert sind, wobei m eine ganze Zahl ist und 0 ≤ m< n ist.

Bei der Erfindung werden also - ebenso wie bei dem bekannten, zuletzt genannten Computertomographen - Messdaten von Strahlen akquiriert, die innerhalb eines (ersten) Messfensters liegen, das - in Richtung der Rotationsachse gemessen - um den Faktor 2n + 1 größer ist als der Abstand zweier benachbarter Windungen der Helix. Bei der Rekonstruktion werden aber Messdaten aus einem zweiten, kleineren und mittig zum ersten Messfenster angeordneten Messfenster mit einem anderen Gewichtungsfaktor beaufschlagt als die außerhalb dieses zweiten Messfensters, aber im ersten Messfenster akquirierten Messdaten.

Die Erfindung basiert auf der Idee, dass man den Untersuchungsbereich sowohl aus den im ersten Messfenster akquirierten Messdaten als auch aus den im zweiten Messfenster akquirierten Messdaten vollständig rekonstruieren kann. Wenn man die durch diese unterschiedlichen Rekonstruktionen erzeugten CT-Bilder zueinander addiert, werden die geschilderten Probleme mehr oder weniger unterdrückt. Es ergibt sich eine geringere Empfindlichkeit gegenüber Abtastfehlern (im Vergleich zu einem Computertomographien mit einem Messfenster zwisdim zwei benachbarten Windungen), und die Bewegungsunschärfe sowie etwaige Artefakte werden reduziert (im Vergleich zu einem Computertomographen mit einem sich über mehrere Windungen der Helix erstreckenden Messfenster). Aufgrund der Linearität des Rekonstruktionsverfahrens ist die Verarbeitung der Messdaten in Abhängig davon, ob sie im zweiten oder nur im ersten Messfenster akquiriert wurden, der gewichteten Summation der beiden CT-Bilder äquivalent, die getrennt aus den CT-Daten des ersten bzw. des zweiten Messfensters rekonstruiert wurden.

Das der Erfindung zugrundeliegende Prinzip läßt sich auch auf mehr als zwei Messfenster ausdehnen, wie in Anspruch 2 angegeben. Voraussetzung dabei ist, dass das erste Messfenster sich in achsialer Richtung über einen Bereich von wenigstens 5p erstreckt (n ≥ 2) und dass das weitere Fenster kleiner ist als das erste und größer oder kleiner als das zweite Fenster.
Die in Anspruch 3 vorgesehene Einstellbarkeit der Gewichte, mit denen die aus den verschiedenen Messfenstern abgeleiteten Messdaten in die Rekonstruktion eingehen, gestattet es, die Gewichte unterschiedlichen Bedingungen im Untersuchungsbereich anzupassen. Wenn die Gefahr der Bewegungsunschärfe groß ist, ist es sinnvoll, die im zweiten Messfenster akquirierten Messdaten stärker zu gewichten. Andernfalls ist eine geringere Betonung dieser Messdaten zweckmäßig.

Grundsätzlich kann man die Messdaten aus beiden Fenstern - von deren unterschiedlichen Gewichtung einmal abgesehen - identisch verarbeiten. Es ist aber auch möglich, gemäß Anspruch 4 die Messdaten in unterschiedlicher Weise zu verarbeiten. Wenn das zweite Messfenster durch benachbarte Windungen der Helix begrenzt wird (m = 0), ist es nämlich möglich, die Filterung der Messdaten aus diesem Fenster so durchzuführen wie in der US-Appln 09/663634 (PHD 99123) erläutert, womit sich eine verbesserte Bildqualität ergibt.

Anspruch 5 beschreibt ein Computerprogramm für die Rekonstruktionseinheit eines Computertomographen, mit dem sich die Erfindung in einem Computertomographen implementieren läßt.

Die Erfindung wird nachstehend anhand der Zeichnung näher erläutert. Es zeigen:
- Fig. 1: einen erfindungsgemäßen Computertomographen,
- Fig. 2: ein Ablaufdiagramm des mit einem solchen Computertomographen durchgeführten Verfahrens,
- Fig. 3: die räumliche Lage der Strahlenquelle und der Randstrahlen des Messfensters in bezug auf die Helix,
- Fig 4: eine Abwicklung der Detektoreinheit und
- Fig. 5: den Verlauf der Linien, entlang derer bei der Rekonstruktion eine eindimensionale Filterung ausgeführt wird.

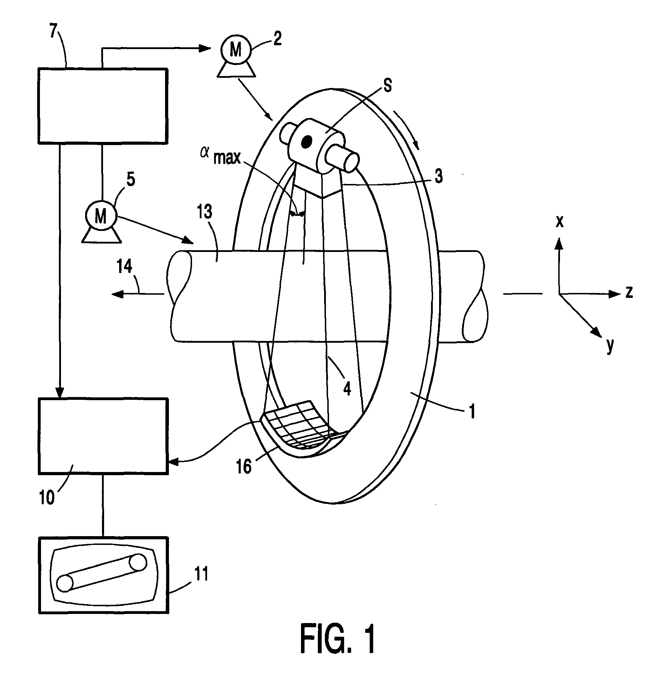

Der in Fig. 1 dargestellte Computertomograph umfasst eine Gantry 1, die um eine parallel zur z-Richtung des in Fig. 1 dargestellten Koordinatensystems verlaufende Rotationsachse 14 rotieren kann. Dazu wird die Gantry von einem Motor 2 mit einer vorzugsweise konstanten Winkelgeschwindigkeit angetrieben. An der Gantry ist eine Strahlenquelle S, beispielsweise eine Röntgenröhre, befestigt. Diese ist mit einer Kollimatoranordnung 3 versehen, die aus der von der Strahlenquelle S erzeugten Strahlung ein kegelförmiges Strahlenbündel 4 ausblendet, d. h. ein Strahlenbündel, das sowohl in Richtung der z-Achse als auch in einer dazu senkrechten Richtung (d. h. in der x-y-Ebene) eine von Null verschiedene endliche Ausdehnung hat.

Das Strahlenbündel durchdringt ein nicht näher dargestelltes Objekt, das sich in einem Untersuchungsbereich 13 befindet. Der Untersuchungsbereich 13 hat die Form eines Zylinders. Nach dem Durchsetzen des Untersuchungsbereichs 13 trifft das Röntgenstrahlenbündel 4 auf eine an der Gantry 1 befestigte Detektoreinheit 16, die eine Anzahl von in z-Richtung gegeneinander versetzten Detektorzeilen umfasst. Jede Detektorzeile befindet sich in einer zur z-Richtung senkrechten Ebene und enthält eine Vielzahl von Detektorelementen, die alle je einen Strahl erfassen und entsprechende Messdaten liefern. Die Detektoreinheit 16 kann auf einem Kreisbogen um die Rotationsachse 14 angeordnet sein; jedoch sind auch andere Detektorgeometrien möglich, z. B. die Anordnung auf einem Kreisbogen um die Strahlenquelle S.

Der mit αₘₐₓ bezeichnete Öffnungswinkel des Strahlenbündels 4 (als Öffnungswinkel ist der Winkel definiert, den ein in der x-y-Ebene am Rande liegender Strahl des Bündels 4 mit einer durch die Strahlenquelle S und die Rationsachse 14 definierten Ebene einschließt) definiert den Durchmesser des Untersuchungsbereiches. Der Untersuchungsbereich 13 - bzw. ein darin befindliches Objekt - beispielsweise ein auf einem Patientenlagerungstisch befindlicher Patient - kann mittels eines Motors 5 parallel zur Richtung der z-Achse verschoben werden. Die Geschwindigkeit dieses Vorschubs in z-Richtung ist konstant und vorzugsweise einstellbar.

Die von der Detektoreinheit 16 akquirierten Messdaten werden einem Bildverarbeitungsrechner 10 zugeführt, der daraus die Absorptionsverteilung in dem vom Strahlenkegel 4 erfassten Teil des Untersuchungsbereichs 13 rekonstruiert und z. B. auf einem Monitor 11 wiedergibt. Die beiden Motoren 2 und 5, der Bildverarbeitungsrechner 10, die Strahlenquelle S und der Transfer der Messdaten von der Detektoreinheit 16 zum Bildverarbeitungsrechner 10 werden von einer geeigneten Kontrolleinheit 7 gesteuert.
Wenn der Motor 5 stillsteht und der Motor 2 die Gantry rotieren lässt, ergibt sich eine kreisförmige Abtastbewegung der Strahlenquelle S und der Detektoreinheit. Die Kontrolleinheit 7 kann die Motoren 2 und 5 aber auch gleichzeitig einschalten, und zwar so, dass das Verhältnis der Vorschubgeschwindigkeit des Untersuchungsbereichs 13 und die Windgeschwindigkeit der Gantry in einem konstanten Verhältnis stehen. In diesem Fall bewegen sich die Strahlenquelle S und der Untersuchungsbereich 13 relativ zueinander auf einer helixförmigen Bahn.

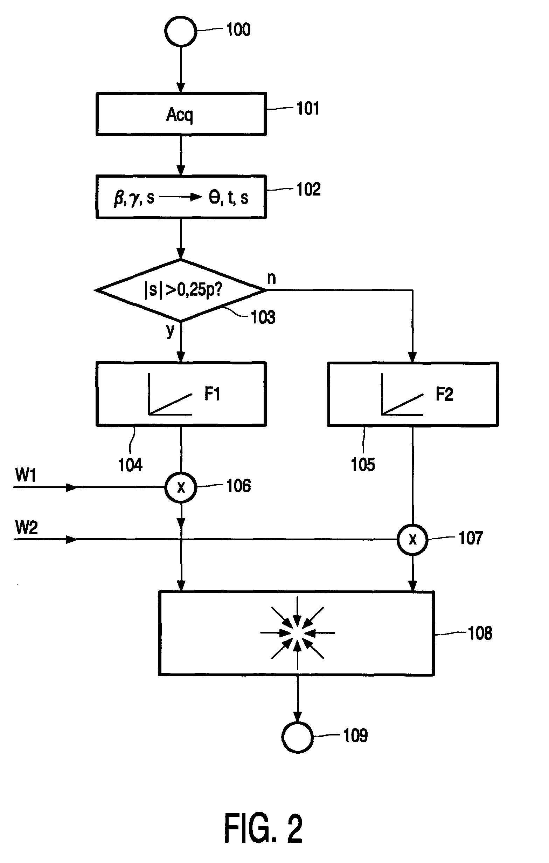

Nachfolgend soll anhand des in Fig. 2 dargestellten Ablaufdiagramms erläutert werden, wie mit dem Computertomographen nach Fig. 1 Messdaten akquiriert und wie aus diesen Messdaten ein CT-Bild rekonstruiert wird.

Nach der Initialisierung im Schritt 100 werden die Motoren 2 und 5 aktiviert und die Strahlenquelle S eingeschaltet. Die daraufhin im Schritt 101 akquirierten, von der Schwächung des Strahlenbündels im Untersuchungsbereich abhängigen Messdaten der Detektoreinheit 16 werden in einem Speicher der Rekonstruktionseinheit 10 transferiert.

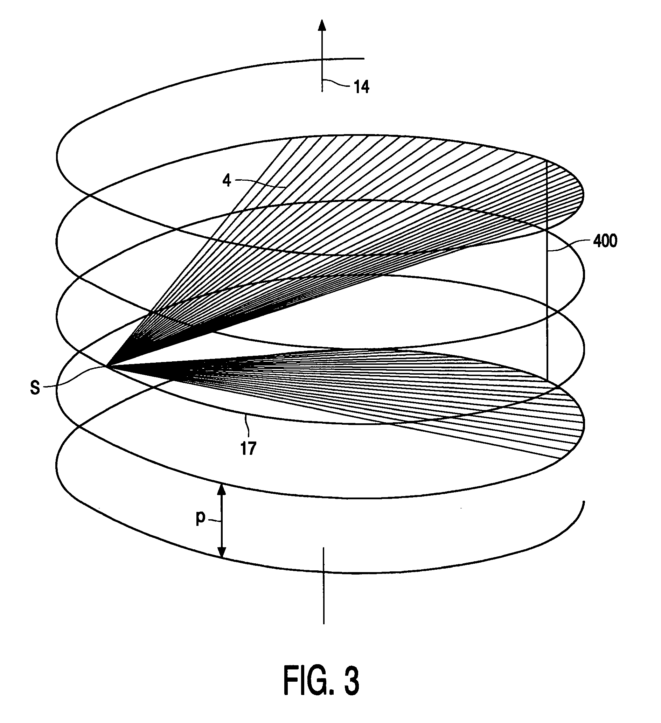

Fig. 3 zeigt die geometrischen Verhältnisse bei der Erfassung der Messdaten. Dabei ist angenommen, dass sich die Strahlenquelle S auf einer Bahn 17 in Form einer Helix um den in Fig. 3 nicht näher dargestellten, stillstehenden Untersuchungsbereich bewegt, obwohl sie tatsächlich nur eine Kreisbewegung ausführt und der Untersuchungsbereich bzw. das zu untersuchende Objekt verschoben wird. Diese Annahme ist aber zulässig, weil es nur auf die Relativbewegung zwischen der Strahlenquelle S und dem Untersuchungsbereich ankommt.

Das für die Rekonstruktion ausgenutzte Strahlenbündel 4 ist auf ein erstes Messfenster beschränkt. Die in der Zeichnung dargestellten, von der Strahlenquelle ausgehenden Geraden bzw. Randstrahlen des Strahlenbündels 4 schneiden die in Richtung der Rotationsachse gegeneinander versetzten Ränder dieses Messfensters und zugleich zwei der Strahlenquelle gegenüberliegende Windungen der Helix. Für die Messung werden also nur solche Strahlen ausgewertet, die mit den gezeichneten Randstrahlen zusammenfallen oder die zwischen diesen Randstrahlen liegen. Das Messfenster liegt symmetrisch zur Strahlenquelle S. Die den Rand des Messfensters definierenden Windungen der Helix haben in Richtung der Rotationsachse 14 den Abstand 3p voneinander, wobei p der Abstand zweier benachbarter Windungen der Helix ist.

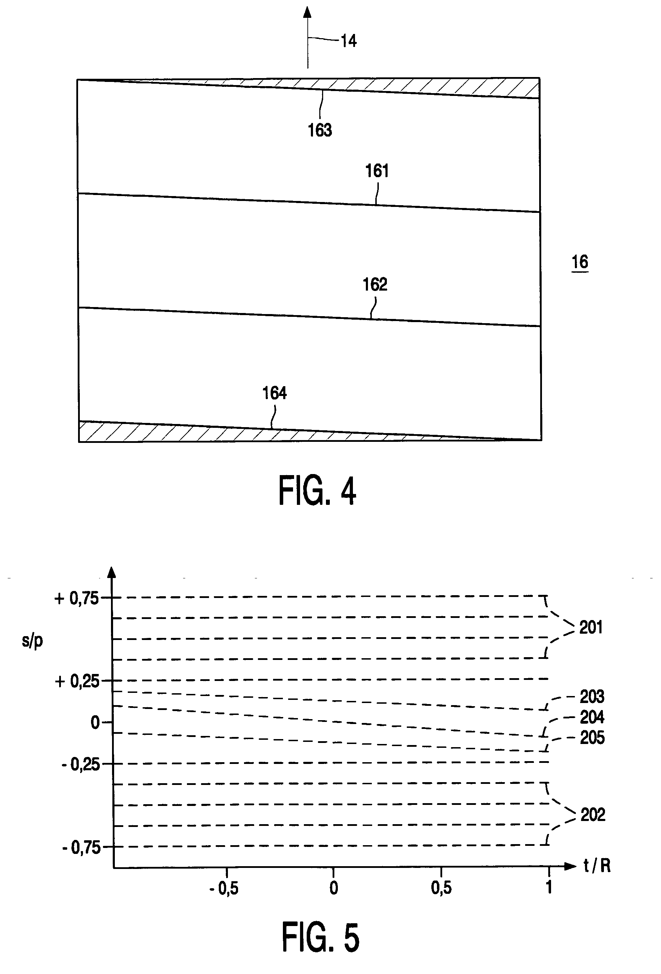

Fig. 4 stellt eine Abwicklung der Detektoreinheit 16 dar, wobei deren Abmessungen in Richtung der Rotationsachse der Deutlichkeit halber in einem wesentlich größeren Maßstab dargestellt sind als in Richtung senkrecht dazu. Mit 163 und 164 sind dabei die in Richtung der Rotationsachse gegeneinander versetzten Ränder des ersten Messfensters auf der Detektoreinheit bezeichnet. Wegen der Steigung der Helix verlaufen diese Ränder nicht horizontal, sondern geneigt. Wenn der Detektor 16 nicht um die Rotationsachse 14, sondern um die Strahlenquelle S gekrümmt wäre oder wenn er eben wäre, wären die Ränder 163 und 164 nicht geradlinig, wie in Fig. 4 dargestellt.

In Fig. 4 ist außerdem ein zweites Messfenster eingezeichnet, dessen in Richtung der Rotationsachse 14 versetzen Ränder mit 161 und 162 bezeichnet sind. Die Ränder werden durch die von der Strahlenquelle S ausgehenden Geraden definiert, die die beiden Windungen im Innern des ersten Messfeldes und gleichzeitig diese Ränder durchstoßen.

Ein beliebiger Punkt im Untersuchungsbereich wird bei seinem Eintritt in das erste Messfenster auf ein Detektorelement projiziert, das z. B. auf dem unteren Rand 164 liegt (in diesem Fall durchstößt der Strahl, auf dem der Punkt liegt, eine Windung der Helix). Im weiteren Verlauf wird dieser Punkt auf ein Detektorelement auf der Linie 162 projiziert (in diesem Fall durchstößt der Strahl durch den Punkt eine benachbarte Windung), die das zweite Messfenster nach unten begrenzt. Nach Durchlaufen eines Bestrahlungsbereichs von weiteren 180° wird dieser Punkt auf ein Detektorelement auf dem oberen Rand 161 des zweiten Messfensters projiziert (wobei der Strahl die nächste Windung durchstößt). Wenn dieser Punkt von der Strahlenquelle S über einen Winkelbereich von insgesamt 540° bestrahlt worden ist, wird er auf ein Detektorelement projiziert, das am oberen Rand 163 des ersten Messfensters liegt (dann schneidet der Strahl die oberste Windung).

Der zu dem ersten Messfenster passende Verlauf des Strahlenbündels 4 läßt sich durch geeignete Ausgestaltung des Kollimators 3 erreichen. Wenn dies nicht möglich ist und das Strahlenbündel 4 den gesamten rechteckigen Bereich des Detektors 16 bestrahlt, erreicht man die Beschränkung auf das Messfenster, indem man die Messdaten von Detektorelementen in dem schraffierten Bereich außerhalb der Linien 163 und 164 nicht zur Rekonstruktion heranzieht.

Die im Schritt 101 akquirierten Messdaten entsprechen - gegebenenfalls nach einer Glättung und einer Logarithmierung - dem Linienintegral der Schwächung entlang des Strahles, entlang dessen sie gemessen wurden. Erforderlichenfalls können in diesem Schritt sämtliche Messdaten mit dem Cosinus des Winkels gewichtet werden, den der zugehörige Strahl mit einer zur Rotationsachse senkrechten Ebene einschließt. Wenn der Kosinus aber für alle Strahlen praktisch den Wert 1 hat (weil der Winkel sehr klein ist), kann diese Gewichtung auch entfallen.

Alle Messdaten M sind durch eine (skalare) Größe gekennzeichnet, die dem Linienintegral der Schwächung entspricht, und durch die Lage der Strahlen, entlang derer sie erfasst worden sind. Jeder Strahl ist durch drei Parameter (β, γ, s) gekennzeichnet:
Der Parameter β kennzeichnet dabei die Richtung eines Lotes von der Strahlenquellenpostion auf die Rotationsachse 14 in einer zur Rotationsachse senkrechten (x,y-)Ebene. Alle Strahlen in dem in Fig. 3 dargestellten Strahlenbündel haben also den gleichen Parameter β. β ist nach mehr als einem Umlauf der Strahlenquelle größer als 2π.
Der Parameter γ ist der Winkel, den der betreffende Strahl in der zur Rotationsachse 14 senkrechten (x,y-) Ebene mit dem erwähnten Lot einschließt. Alle Strahlen innerhalb eines zur Rotationsachse parallelen Strahlenfächers haben denselben Wert γ. In Fig. 3 ist ein solcher Strahlenfächer durch die Gerade 400 und die (Rand-)Strahlen definiert, die diese Gerade mit der Strahlenquelle S verbindenden.
Der Parameter s stellt die Höhenkoordinate des Strahles dar, d. h. er gibt an, in welchem Lage der betreffende Strahl zwischen zwei Windungen der Helix verläuft. Alle Strahlen, die dieselbe Windung der Helix schneiden, haben den gleichen Wert s. Die Randstrahlen des ersten Messfensters sind durch den Parameter s = ± 0,75 p gekennzeichnet; die Randstrahlen des zweiten Messfensters haben den Parameter s = ± 0,25 p

Somit ist jeder Strahl durch einen Punkt in dem dreidimensionalen (β, γ, s) -Parameterraum gekennzeichnet. Die Akquisition der CT-Daten stellt daher eine Abtastung der sogenannten Objektfunktion (in diesem Fall des Linienintegrals der Schwächung) an einer Vielzahl von relativ gleichmäßig im (β, γ, s) -Parameterraum verteilten Abtastpunkten dar. Die Abtastung in diesem Parameterraum ist für die weitere Verarbeitung jedoch noch nicht optimal geeignet.

Deshalb erfolgt im Schritt 102 ein sogenanntes "Rebinning" in eine Parallelstrahlgeometrie. Dabei wird durch Umsortierung und Uminterpolation aus den akquirierten Messdaten M(β, γ, s) ein Datensatz M(θ, t, s) berechnet, der die Objektfunktion auf den Gitterpunkten eines regelmäßigen kartesischen Gitters in einem dreidimensionalen (θ, t, s) - Parameterraum darstellt:
Der Parameter θ bezeichnet dabei die Richtung eines zur Rotationsachse parallelen Strahlenfächers in einer zur Rotationsachse senkrechten Ebene. Die Strahlen in zur Rotationsachse und zueinander parallelen Strahlenfächern haben denselben meter θ. Ebenso wie der Parameter β kann auch der Parameter θ größer werden als 2π.
Der Parameter t bezeichnet den Abstand eines Strahlenfächers von der Rotationsachse, wobei die auf der einen Seite der Rotationsachse befindlichen Strahlenfächer einen negativen und die auf der anderen Seite befindlichen einen positiven Wert von t haben. Der Maximalwert von t entspricht dem Radius des Untersuchungsbereichs 13.
Der Parameter s ist wiederum die Höhenkoordinate.

Wenn man einmal von der Verwendung eines zweiten Messfensters absieht, ist das insoweit beschriebene Verfahren aus der eingangs erwähnten US-Appln 09/368850 bekannt. Während aber bei dem bekannten Verfahren die weitere Verarbeitung der Messdaten unabhängig davon erfolgt, wo sie innerhalb des (ersten) Mesfensters verlaufen, werden die Messdaten bei der Erfindung in Abhängigkeit davon weiterverarbeitet, ob sie zu Strahlen gehören, die auch durch das zweite Messfenster verlaufen oder nicht.

Dementsprechend erfolgt im Schritt 103 eine Abfrage, ob der Wert s der Messdaten M(θ, t, s) dem Betrage nach größer ist als 0,25p (wobei p dem Abstand benachbarter Windungen der Helix entspricht). Wenn dies zutrifft, verlaufen die zu diesen Messdaten gehörenden Strahlen zwar innerhalb des ersten Messfensters aber außerhalb des zweiten Messfensters. In diesem Fall erfolgt im Schritt 104 eine eindimensionale Filterung über alle CT-Daten, die den gleichen Wert von θ und s haben, aber unterschiedliche Werte t.

Diese Filterung ist in Fig. 5 veranschaulicht. Fig. 5 stellt eine zur θ-Achse parallele Ebene im kartesischen (θ, t, s) Parameterraum dar, mit dem (auf den Wert p normierten) Parameter s als Ordinate und dem (auf den Radius R des Untersuchungsbereichs normierten) Parameter t als Abszisse. Die gestrichelt bzw. strichpunktiert angedeuteten Linien verbinden die aufeinanderfolgenden Gitterpunkte bzw. Messdaten, die einer gemeinsamen Filterung unterzogen werden. Diese Linien verlaufen für alle Messdaten, bei denen der Wert s zwischen 0,25p und 0,75p sowie zwischen -0,75p und -0,25p liegt, horizontal, wie etwa bei den Linien 201 und 202.

Alle Messdaten, die innerhalb des zweiten Messfensters liegen (d. h. deren Parameter s nicht größer ist als 0,25p und nicht kleiner als -0,25p), werden im Block 105 einer Filterung unterzogen, wobei jedoch nicht die Messdaten einer eindimensionalen Filterung unterzogen werden, die auf einer horizontalen Linie liegen, sondern Messdaten von Strahlen, die in Fig. 5 durch mehr oder weniger schräg verlaufende Geraden 203, 204 und 205 miteinander verbunden sind Dieses Filterverfahren ist im einzelnen in der US-Appln. 09/63634 erläutert, worauf ausdrücklich Bezug genommen wird. Durch diese Filterung ergibt sich eine gewisse Verbesserung der Bildqualität.
Die außerhalb des zweiten Messfensters (aber innerhalb des ersten Messfensters) akquirierten Messdaten werden mit einem Gewichtungsfaktor w₁ gewichtet (Block 106) und die innerhalb des zweiten Messfensters akquirierten, in Schritt 105 gefilterten Messdaten mit einem zweiten Gewichtungsfaktor w₂ (Block 107). Aus den so gefilterten und gewichteten Messdaten wird im Schritt 108 durch eine sogenannte Rückprojektion die Schwächung in den einzelnen Punkten des Untersuchungsbereichs ermittelt. Für jeden Punkt im Untersuchungsbereich werden dabei die Strahlen ermittelt, die diesen Punkt des Untersuchungsbereichs aus einem Winkelbereich von 3π durchstrahlt haben. Die zu diesen Strahlen gehörigen Messdaten werden - erforderlichenfalls nach einer weiteren Interpolation - mit w₁ bzw. w₂ gewichtet und summiert. Das so rekonstruierte Bild wird auf geeignete Weise wiedergegeben und gespeichert. Danach ist das Verfahren beendet (Block 109).

Die Gewichtungsfaktoren w₁ und w₂ können interaktiv vom Benutzer vorgegeben werden. Sie können aber auch in Abhängigkeit von der abzubildenden Körperregion automatisch vorgegeben werden.

Wenn der Gewichtungsfaktor w₁ = 0 (oder klein im Vergleich zu w₂) ist, werden nur Messdaten aus dem zweiten Messfenster zur Rekonstruktion herangezogen. Dadurch ergibt sich ein CT-Bild mit geringer Bewegungsunschärfe, aber einer erhöhten Empfindlichkeit gegenüber Abtastfehlern. Wenn der Gewichtungsfaktor w₂ doppelt so groß gewählt wind wie der Gewichtungsfaktor w₁, ergeben sich aufgrund der Linearität des Rekonstruktionsverfahrens dieselben Verhältnisse als würde man ein erstes CT-Bild aus sämtlichen im ersten Fenster akquirierten Messdaten rekonstruieren und dieses zu einem zweiten CT-Bild addieren, das lediglich aus den im zweiten Messfenster akquirierten Messdaten rekonstruiert wird. Das Signal/Rauschverhältnis ist dabei besser und die Wirkung von Abtastfehlern ist reduziert, doch wird dieser Vorteil dadurch erkauft, dass etwaige Bewegungsunschärfen stärker hervortreten.

Wenn die Gewichtungsfaktoren w₁ und w₂ gleich groß sind, dann erhält man im wesentlichen dieselben Verhältnisse als wenn man aus allen im ersten Messfenster akquirierten Messdaten ein gleichmäßig gewichtetes CT-Bild rekonstruieren würde. Das Signal/Rauschverhältnis ist dann optimal, doch ist auch die Gefahr von Bewegungsunschärfen noch größer.
Die Messdaten aus dem zweiten Messfenster können, abweichend von der Darstellung in der Fig. 2, auch auf dieselbe Weise gefiltert werden, wie die Messdaten im ersten Messfenster, d. h. entlang paralleler Linien in der t, s-Ebene (Fig. 5). Die Verzweigung 103 muss dann erst nach der einheitlichen Filterung vorgenommen werden. Dabei müseen geringe Abstriche bei der Bildqualität in Kauf genommen werden.

Vorstehend wurde davon ausgegangen, dass die Abmessungen des ersten Messfensters in Richtung der Rotationsachse dem dreifachen Windungsabstand p der Helix entsprechen. Die Abmessungen des Messfensters können aber auch 3p, 5p oder allgemein (2n +1)p betragen, wobei n eine ganze Zahl ist. Man kann dann wenigstens zwei weitere Messfenster mit den Abmessungen (2m + 1)p und (2k + 1)p definieren (und gegebenenfalls noch weitere Messfenster), wobei m und k voneinander verschiedene positive ganze Zahlen sind, die kleiner sind als n. Die Messdaten aus diesen verschiedenen Messfenstern können mit unterschiedlichen Gewichtungsfaktoren in die Rekonstruktion eingehen.

Wie vorstehend bereits erläutert, erreicht man dieselben Ergebnisse, wenn man aus allen in jeweils einem Messfenster akquirierten Messdaten ein CT-Bild rekonstruiert und dieses zu dem CT-Bild bzw. zu den CT-Bildern addiert, die sich aus den Messdaten des oder der anderen Messfenster rekonstruieren lassen.

## Patentansprüche

1. Computertomograph mit
- einer Abtasteinheit, die eine Strahlenquelle (S) und eine damit verbundene Detektoreinheit (16) zur Erfassung eines von der Strahlenquelle emittierten, kegelförmigen Strahlenbündels nach dem Durchgang durch einen Untersuchungsbereich (13) bzw. durch ein darin befindliches Objekt umfasst,
- einer Antriebsanordnung (2,5) zur Erzeugung einer eine Rotation um eine Rotationsachse (14) und einen Vorschub in Richtung parallel zur Rotationsachse umfassenden Relativbewegung in Form einer Helix (17) zwischen der Abtasteinheit (S,16) und dem Untersuchungsbereich (13) bzw. dem Objekt,
- wobei die Helix und die Detektoreinheit so bemessen sind, dass die Detektoreinheit gleichzeitig sämtliche Strahlen des Strahlenbündels innerhalb eines ersten Messfensters erfast, dessen in Richtung der Rotationsachse (14) gegeneinander versetzte Ränder (163, 164) durch von der Strahlenquelle (S) ausgehende und zwei in Richtung der Rotationsachse um die Strecke (2n+1)p versetzte Abschnitte der Helix (17) schneidende Geraden definiert sind , wobei n eine ganze Zahl ≥ 1 ist und p dem achsialen Versatz zweier benachbarter Windungen der Helix entspricht,
- und mit einer Rekonstruktionseinheit (10) zur Rekonstruktion eines der räumlichen Verteilung der Absorption innerhalb des Untersuchungsbereiches (13) entsprechenden CT-Bildes aus den von der Detektoreinheit (16) innerhalb des ersten (160) Messfensters akquirierten Messdaten,
- wobei die Messdaten, die aus Strahlen abgeleitet sind, die innerhalb eines zweiten Messfensters verlaufen, mit einem anderen Gewicht in die Rekonstruktion eingehen als die Messdaten von außerhalb des zweiten Messfensters - aber innerhalb des ersten Messfensters verlaufenden Strahlen und
wobei in Richtung der Rotationsachse gesehen das zweite Messfenster sich in der Mitte des ersten Messfensters befindet und seine gegeneinander versetzten Ränder (163, 164) durch von der Strahlenquelle (S) ausgehende und zwei in Richtung der Rotationsachse um die Strecke (2m+1)p versetzte Abschnitte der Helix (17) schneidende Geraden definiert sind , wobei m eine ganze Zahl ist und 0 ≤ m < n ist.

2. Computertomograph nach Anspruch 1, wobei die Messdaten, die aus Strahlen abgeleitet sind, die innerhalb wenigstens eines zusätzlichen Messfensters verlaufen, mit einem anderen Gewicht in die Rekonstruktion eingehen als die Messdaten von außerhalb des weiteren Messfensters - aber innerhalb des ersten Messfensters verlaufenden Strahlen und wobei in Richtung der Rotationsachse gesehen das zusätzliches Messfenster sich in der Mitte des ersten Messfensters befindet und seine gegeneinander versetzten Ränder (163, 164) durch von der Strahlenquelle (S) ausgehende und zwei in Richtung der Rotationsachse um die Strecke (2k+1)p versetzte Abschnitte der Helix (17) schneidende Geraden definiert sind , wobei k eine ganze Zahl ist, und 0 ≤ k < n und k ≠ m ist.

3. Computertomograph nach Anspruch 1, wobei die Gewichte, mit denen die von Strahlen aus unterschiedlichen Messfenstern abgeleiteten Messdaten eingehen, einstellbar sind.

4. Computertomograph nach Anspruch 1, wobei m=0 ist und die Rekonstruktion eine eindimensionale Filterungen der Messdaten sowie eine Rückprojektion der gefilterten Daten umfasst, und wobei die Filterung der zum ersten Messfenster gehörenden Messdaten von der Filterung der zum ersten Messfenster gehörenden Messdaten abweicht.

5. Computerprogramm für die Rekonstruktionseinheit eines Computertomographen, der versehen ist mit
- einer Abtasteinheit, die eine Strahlenquelle (S) und eine damit verbundene Detektoreinheit (16) zur Erfassung eines von der Strahlenquelle emittierten, kegelförmigen Strahlenbündels nach dem Durchgang durch einen Untersuchungsbereich (13) bzw. durch ein darin befindliches Objekt umfasst,
- einer Antriebsanordnung (2,5) zur Erzeugung einer eine Rotation um eine Rotationsachse (14) und einen Vorschub in Richtung parallel zur Rotationsachse umfassenden Relativbewegung in Form einer Helix (17) zwischen der Abtasteinheit (S,16) und dem Untersuchungsbereich (13) bzw. dem Objekt,
- wobei die Helix und die Detektoreinheit so bemessen sind, dass die Detektoreinheit gleichzeitig sämtliche Strahlen des Strahlenbündels innerhalb eines ersten Messfensters erfasst, dessen in Richtung der Rotationsachse (14) gegeneinander versetzte Ränder (163, 164) durch von der Strahlenquelle (S) ausgehende und zwei in Richtung der Rotationsachse um die Strecke (2n+1)p versetzte Abschnitte der Helix (17) schneidende Geraden definiert sind , wobei n eine ganze Zahl ≥ 1 ist und p dem achsialen Versatz zweier benachbarter Windungen der Helix entspricht,
wobei die Rekonstruktionseinheit (10) zur Rekonstruktion eines der räumlichen Verteilung der Absorption innerhalb des Untersuchungsbereiches (13) entsprechenden CT-Bildes aus den von der Detektoreinheit (16) innerhalb des ersten (160) Messfensters akquirierten Messdaten dient,
- wobei die Messdaten, die aus Strahlen abgeleitet sind, die innerhalb eines zweiten Messfensters verlaufen, mit einem anderen Gewicht in die Rekonstruktion eingehen als die Messdaten von außerhalb des zweiten Messfensters - aber innerhalb des ersten Messfensters verlaufenden Strahlen und
wobei in Richtung der Rotationsachse gesehen das zweite Messfenster sich in der Mitte des ersten Messfensters befindet und seine gegeneinander versetzten Ränder (163, 164) durch von der Strahlenquelle (S) ausgehende und zwei in Richtung der Rotationsachse um die Strecke (2m+1)p versetzte Abschnitte der Helix (17) schneidende Geraden definiert sind , wobei m eine ganze Zahl ist und 0 ≤ m < n ist.

## Claims

1. A computer tomograph which includes
- a scanning unit which includes a radiation source (S) and a detector unit (16) connected thereto in order to detect a conical radiation beam emitted by the radiation source, after the passage of the beam through an examination zone (13) or an object present therein,
- a driving device (2, 5) for producing a relative movement in the form of a helix (17), consisting of a rotation about an axis of rotation (14) and an advance in the direction parallel to the axis of rotation, between the scanning unit (S, 16) and the examination zone (13) or the object,
- the helix and the detector unit being proportioned in such a manner that the detector unit simultaneously detects all rays of the radiation beam within a first measuring window whose edges (163, 164), being offset relative to each other in the direction of the axis of rotation (14), are defined by lines which originate from the radiation source (S) and intersect two segments of the helix (17) which are offset over the distance (2n + 1)p in the direction of the axis of rotation, where n is an integer number ≥ 1 and p corresponds to the axial offset of two neighboring turns of the helix,
- and a reconstruction unit (10) for reconstructing a CT image which corresponds to the spatial distribution of the absorption within the examination zone (13) from the measuring data acquired by the detector unit (16) within the first measuring window (160),
- the measuring data derived from rays which extend within a second window being assigned a weight for the reconstruction that differs from that assigned to the measuring data derived from rays which extend outside the second measuring window but inside the first measuring window, and
- the second window being situated, viewed in the direction of the axis of rotation, at the center of the first measuring window and its mutually offset edges (163, 164) being defined by lines which originate from the radiation source (S) and intersect two segments of the helix (17) which are offset over the distance (2m + 1)p in the direction of the axis of rotation, where m is an integer number and 0 ≤ m ≤ n.

2. A computer tomograph as claimed in claim 1, wherein the measuring data derived from rays which extend within at least one additional measuring window enter the reconstruction with a weight that differs from that of the measuring data from rays which extend outside the second measuring window but within the first measuring window, and wherein, viewed in the direction of the axis of rotation, the additional measuring window is situated at the center of the first measuring window and its mutually offset edges (163, 164) are defined by lines which originate from the radiation source (S) and intersect two segments of the helix (17) which are offset over the distance (2k + 1)p in the direction of the axis of rotation, where k is an integer number and 0 ≤ k < n and k # m.

3. A computer tomograph as claimed in claim 1, wherein the weights which are assigned to the measuring data derived from rays from different measuring windows are adjustable.

4. A computer tomograph as claimed in claim 1, wherein m = 0 and the reconstruction includes one-dimensional filtering of the measuring data as well as back projection of the filtered data, the filtering of the measuring data associated with the first measuring window being different from the filtering of the measuring data associated with the second measuring window.

5. A computer program for the reconstruction unit of a computer tomograph which includes
- a scanning unit which includes a radiation source (S) and a detector unit (16) which is connected thereto in order to detect a conical radiation beam, emitted by the radiation source, after its passage through an examination zone (13) or an object present therein,
- a driving device (2, 5) for producing a relative movement in the form of a helix (17), consisting of a rotation about an axis of rotation (14) and an advance in the direction parallel to the axis of rotation, between the scanning unit (S, 16) and the examination zone (13) or the object,
- the helix and the detector unit being proportioned in such a manner that the detector unit simultaneously detects all rays of the radiation beam within a first measuring window whose edges (163, 164), being offset relative to each other in the direction of the axis of rotation (14), are defined by lines which originate from the radiation source (S) and intersect two segments of the helix (17) which are offset over the distance (2n + 1)p in the direction of the axis of rotation, where n is an integer number ≥ 1 and p corresponds to the axial offset of two neighboring turns of the helix, and the reconstruction unit (10) serving for the reconstruction of a CT image corresponding to the spatial distribution of the absorption within the examination zone (13), from the measuring data acquired by the detector unit (16) within the first measuring window (160),
- the measuring data derived from rays which extend within a second measuring window being assigned a weight for the reconstruction that differs from that of measuring data derived from rays which extend outside the second measuring window but inside the first measuring window, and
- the second measuring window being situated, viewed in the direction of the axis of rotation, at the center of the first measuring window and its mutually offset edges (163, 164) being defined by lines which originate from the radiation source (S) and intersect two segments of the helix (17) which are offset over the distance (2m + 1)p in the direction of the axis of rotation, where m is an integer number and 0 ≤ m < n.

## Revendications

1. Scanner CT comprenant
- une unité de balayage qui comporte une source de rayons (S) et une unité de détection (16) reliée à cette source pour détecter un faisceau de rayons conique émis par la source de rayons, après le passage à travers une zone d'examen (13) ou à travers un objet se trouvant dans cette zone,
- un dispositif d'entraînement (2,5) pour produire un mouvement relatif en forme d'hélice (17) comportant une rotation autour d'un axe de rotation (14) et un avancement dans un sens parallèle à l'axe de rotation, entre l'unité de balayage (S, 16) et la zone d'examen (13) ou l'objet,
- l'hélice et l'unité de détection étant dimensionnées de telle sorte que l'unité de détection détecte simultanément tous les rayons du faisceau de rayons à l'intérieur d'une première fenêtre de mesure, dont les bords (163, 164) décalés l'un par rapport à l'autre, dans le sens de l'axe de rotation (14), sont définis par des droites qui partent de la source de rayons (S) et coupent deux segments de l'hélice (17) décalés, dans le sens de l'axe de rotation, de la distance (2n + 1)p, n étant un nombre entier ≥ 1 et p correspondant au décalage axial de deux spires voisines de l'hélice,
- et une unité de reconstruction (10) pour la reconstruction d'une image CT correspondant à la répartition spatiale de l'absorption à l'intérieur de la zone d'examen (13) à partir des données de mesure acquises par l'unité de détection (16) à l'intérieur de la première fenêtre de mesure (160),
- les données de mesure, qui sont dérivées de rayons qui passent à l'intérieur d'une seconde fenêtre de mesure, intervenant dans la reconstruction avec un autre poids que les données de mesure de rayons passant à l'extérieur de la seconde fenêtre de mesure mais à l'intérieur de la première fenêtre de mesure, et
- vu dans le sens de l'axe de rotation, la deuxième fenêtre de mesure se trouvant au milieu de la première fenêtre de mesure et ses bords (163, 164) décalés l'un par rapport à l'autre étant définis par des droites qui partent de la source de rayons (S) et coupent deux segments de l'hélice (17) décalés, dans le sens de l'axe de rotation, de la distance (2m + 1)p, m étant un nombre entier et 0 ≤ m < n.

2. Scanner CT suivant la revendication 1, dans lequel les données de mesure, qui sont dérivées de rayons qui passent à l'intérieur d'au moins une fenêtre de mesure supplémentaire, interviennent dans la reconstruction avec un autre poids que les données de mesure de rayons passant à l'extérieur de l'autre fenêtre de mesure mais à l'intérieur de la première fenêtre de mesure et dans lequel, vu dans le sens de l'axe de rotation, la fenêtre de mesure supplémentaire se trouve au milieu de la première fenêtre de mesure et ses bords (163, 164) décalés l'un par rapport à l'autre sont définis par des droites qui partent de la source de rayons (S) et coupent deux segments de l'hélice (17) décalés, dans le sens de l'axe de rotation, de la distance (2k + 1)p, k étant un nombre entier et 0 ≤ k < n et k ≠ m.

3. Scanner CT suivant la revendication 1, dans lequel les poids avec lesquels les données de mesure dérivées de rayons provenant de différentes fenêtres de mesure interviennent, sont réglables.

4. Scanner CT suivant la revendication 1, dans lequel m = 0 et la reconstruction comprend des filtrages unidimensionnels des données de mesure ainsi qu'une rétroprojection des données filtrées, et dans lequel le filtrage des données de mesure appartenant à la première fenêtre de mesure diffère du filtrage des données de mesure appartenant à la deuxième fenêtre de mesure.

5. Programme informatique pour l'unité de reconstruction d'un scanner CT, qui est doté de:
- une unité de balayage qui comporte une source de rayons (S) et une unité de détection (16) reliée à cette source pour détecter un faisceau de rayons conique émis par la source de rayons, après le passage à travers une zone d'examen (13) ou à travers un objet se trouvant dans cette zone,
- un dispositif d'entraînement (2,5) pour produire un mouvement relatif en forme d'hélice (17) comportant une rotation autour d'un axe de rotation (14) et un avancement dans un sens parallèle à l'axe de rotation, entre l'unité de balayage (5, 16) et la zone d'examen (13) ou l'objet,
- l'hélice et l'unité de détection étant dimensionnées de telle sorte que l'unité de détection détecte simultanément tous les rayons du faisceau de rayons à l'intérieur d'une première fenêtre de mesure, dont les bords (163, 164) décalés l'un par rapport à l'autre, dans le sens de l'axe de rotation (14), sont définis par des droites qui partent de la source de rayons (S) et coupent deux segments de l'hélice (17) décalés, dans le sens de l'axe de rotation, de la distance (2n + 1)p, n étant un nombre entier ≥ 1 et p correspondant au décalage axial de deux spires voisines de l'hélice,
- l'unité de reconstruction (10) servant à la reconstruction d'une image CT correspondant à la répartition spatiale de l'absorption à l'intérieur de la zone d'examen (13) à partir des données de mesure acquises par l'unité de détection (16) à l'intérieur de la première fenêtre de mesure (160),
- les données de mesure, qui sont dérivées de rayons qui passent à l'intérieur d'une seconde fenêtre de mesure, intervenant dans la reconstruction avec un autre poids que les données de mesure de rayons passant à l'extérieur de la seconde fenêtre de mesure mais à l'intérieur de la première fenêtre de mesure, et
- vu dans le sens de l'axe de rotation, la deuxième fenêtre de mesure se trouvant au milieu de la première fenêtre de mesure et ses bords (163, 164) décalés l'un par rapport à l'autre étant définis par des droites qui partent de la source de rayons (S) et coupent deux segments de l'hélice (17) décalés, dans le sens de l'axe de rotation, de la distance (2m + 1)p , m étant un nombre entier et 0 ≤ m < n.
